# EUROPEAN PATENT APPLICATION

(11) **EP 1 340 496 A1**
(43) Date of publication of application: **03.09.2003**
(21) Application number: 01976871.2
(22) Date of filing: 30.10.2001
(51) Int. Cl.: A61K 9/70, A61K 47/36, A61K 47/32, A61K 47/12

(54) **PHARMACEUTICAL PREPARATION OF PERCUTANEOUS ABSORPTION TYPE**

(30) Priority: 07.11.2000 JP 2000339524
(71) Applicant: HISAMITSU PHARMACEUTICAL CO. INC., Tosu-shi, Saga-ken 841-0017 (JP)
(72) Inventor: TERAHARA, Takaaki, Tsukuba Laboratory, Tsukuba-shi, Ibaraki 305-0856 (JP); AIDA, Kazunosuke, Tsukuba Laboratory, Tsukuba-shi, Ibaraki 305-0856 (JP); HIGO, Naruhito, Tsukuba Laboratory, Tsukuba-shi, Ibaraki 305-0856 (JP); SATO, Shuji, Tsukuba Laboratory, Tsukuba-shi, Ibaraki 305-0856 (JP)
(74) Representative: Cresswell, Thomas Anthony
(86) International application number: JP0109496
(87) International publication number: WO02038139

(57) **Abstract**

An adhesive pharmaceutical preparation of the percutaneous absorption type containing an acid addition salt of a basic drug or amphoteric drug, in which the medicinal component highly permeates the skin and which is reduced in skin irritation and excellent in physical stability. The preparation comprises an aminated polymer, a drug in the form of an acid addition salt, and a carboxylic acid or/and a salt thereof, and is characterized in that the content of the aminated polymer is up to 50 wt.% based on the whole preparation, the amount of the amino groups contained in the polymer is 0.5 mol or higher per mol of the drug, and the amount of the carboxylic acid or/and salt thereof is 1 to 10 mol per mol of the sum of the drug and the amino groups contained in the polymer.

## Description

### Technical Field

The present invention relates to a transdermal preparation for the purpose of percutaneous absorption of drugs. Particularly, the invention relates to adhesive pharmaceutical preparation in which the medicinal component highly permeates the skin, and which is reduced in skin irritation and excellent in physical stability.

### Background of the Invention

Oral administration methods using tablets, capsules, syrups and the like have been employed for numerous drugs as the administration methods of drugs. However, in the cases of oral administration, a first-pass effect in liver easily occurs after absorption of the drug, and serum levels more than needs are transiently observed after the administration. Thus, there has been defect that side effects easily occur. In order to dissolve such defects in the oral administration, development of transdermal preparations has been positively carried forward. It is known that these transdermal preparations cover these defects, in addition, advantages are anticipated such as reduced dosage frequencies, improvement of compliance, and easiness of administration and discontinuation thereof, and they are useful particularly for elderly and child patients.

However, since the normal skin primarily has a barrier function to prevent foreign matters from invading into the body, it is often difficult for bases usually used for the transdermal preparations to provide sufficient percutaneous absorption of medicinal components formulated. Also, since stratum corneum having the barrier function is highly lipophilic, generally permeability of ionic drugs such as drug salts is extremely low.

Thus, designs to enhance percutaneous absorbability of the drugs through the stratum corneum are required, and the ionic drug is generally used in non-ionized form of a drug. For example, Tulobuterol, a bronchodilator which has been used in the form of hydrochloride as the oral drug, is authorized improvement of its percutaneous absorbability by being made into the non-ionized form(WO 97/14411), and in fact, its preparation is on the market. However, lots of basic drugs are problematic in that the drug in the non-ionized form is unstable and stabilizers such as antioxidant have skin irritation. Therefore, the development of the transdermal preparation only by making the basic drug the non-ionized form is problematic for many drugs. Also, in order to solve the problem, attempts to improve the skin permeability have been carried out in which highly lipophilic ion pairs are formed by the use of a low irritant organic acid for counterions. For instance, already proposed are the process in which aliphatic carboxylic acid, aromatic carboxylic acid or an inorganic salt thereof is formulated with a basic drug salt (WO 96/16642), the process in which sodium acetate is contained in ketotifen fumarate (JP-A-8-157365), and the process in which sodium acetate is contained in fentanyl citrate (JP-A-10-45570). However, the formation of ion pairs between the basic drug and the organic acid is often dependent on ionic species and properties of the basic drug and the organic acid, and the incomplete formation of ion pairs can occur in some combinations.

Also, a technique in which the drug is combined with a polymer compound having amino groups is reported as a transdermal preparation. For instance, reported are the pharmaceutical preparation of tape in which a natural rubber adhesive is combined with Piroxicam and Eudragit E (methyl methacrylate-butyl methacrylate-dimethylaminoethyl methacrylate copolymer) (JP-2857882), the pharmaceutical preparation of tape using Eudragit E as a preparation basis and containing acrylate having acid groups, an emollient, and the drug (JP-A-10-182439), and the pharmaceutical preparation of tape using Eudragit E as the preparation basis and containing organic di- or tri-carboxylic acid, an emollient and the drug (JP-A-10-182440). However, purposes of the use of the above Eudragit E are the improvement of drug solubility and enhancement of adherability. Desalt of the basic drug salt, formation promotion of ion pairs between the drug and the organic acid, and enhancement of the skin permeability of physiologically active substances thereby are not included in the purposes.

Also, reported is the transdermal preparation containing the polymer containing basic nitrogen as a means to neutralize an acid addition salt of the drug (JP-2977254), but this invention is aimed to desalt the drug and enhance the solubility thereby. Thus, in the case where stability of the drug in the non-ionized form is problematic, this is poor in practical application.

### Disclosure of the Invention

The present invention was carried out to solve the problems in the prior art described above. The present invention is aimed to provide a transdermal preparation comprising an acid addition salt of a basic drug or amphoteric drug, an adhesive pharmaceutical preparation in which the medicinal component highly permeates the skin and which is reduced in skin irritation and excellent in physical stability.

### Best Mode for Carrying Out the Invention

During an intensive study to solve the above problems, the inventors have found to be capable of providing adhesive pharmaceutical preparation wherein the skin permeability of a drug is significantly enhanced, further it is safe for the skin that is an administration site and excellent in content stability and physical stability of a basis, by containing a polymer compound having amino groups at equal to or greater than the certain amount in adhesive pharmaceutical preparation containing a drug having basic functional groups in a salt type , thereby being converted to the drug in a non-ionized form which enhances its skin permeability, and further by adding an appropriate organic acid thereto thereby efficiently forming ion pairs which enhance a distribution rate to the skin, and have completed the present invention.

That is, the invention relates to the transdermal preparation containing a polymer compound having amino groups, a drug forming an acid addition salt, and carboxylic acid and/or a salt thereof, characterized in that the content of the polymer compound having amino groups is 50% or less by weight based on the whole preparation, and a molar ratio of the amino groups in the polymer compound is 0.5 mol or higher per mol of the drug, and the content of the carboxylic acid and/or the salt thereof is 1 to 10 mol per mol of the sum of the drug and the amino groups in the polymer compound.

Also, the invention relates to methods for improving skin permeability and reducing skin irritation of the transdermal preparation comprising a drug forming an acid addition salt, characterized in that a polymer compound having amino groups, and carboxylic acid and/or a salt thereof are contained.

The amino group in the polymer compound having amino groups used in the invention may be any of primary, secondary and tertiary amino groups.

Examples of the polymer compound having amino groups used in the invention include, for example, polymer compounds having the primary amino groups such as polyaminostylene, polyvinylamine and chitosan, for example, polymers of dialkylaminoalkyl (meth)acrylate such as dimethylaminoethyl (meth)acrylate, diethylaminoethyl (meth)acrylate and dibutylaminoethyl (meth)acrylate, or copolymers between these monomers or one or more of these monomer and the other copolymerizable monomer [e.g., methyl (meth)acrylate, ethyl (meth)acrylate, propyl (meth)acrylate, butyl (meth)acrylate, 2-hydroxyethyl (meth)acrylate, 2-ethylhexyl (meth)acrylate, and the like], polyvinyl acetal dialkylamino acetate such as polyvinyl acetal dimethylamino acetate, polyvinyl acetal diethylamino acetate and polyvinyl acetal dibutylamino acetate, polymer compounds having tertiary amino groups such as polyvinyl pyridine. These polymers may be those crosslinked using an appropriate crosslinker. Even those copolymerized monomers having amino groups in an acryl adhesive used as the basis of the transdermal preparation can be used.

Among these polymer compounds, more preferred are chitosan, polyvinyl acetal diethylamino acetate, and methyl methacrylate-butyl methacrylate-dimethylaminoethyl methacrylate copolymer (brand name: Eudragit E, Rohm GmbH), and in particular, Eudragit E (brand name, Rohm GmbH)is preferable.

These polymer compounds having amino groups may be used alone or in combination with two or more.

It is desirable to formulate such that the amount of use of the polymer compound having amino groups is 50% or less by weight based on the whole preparation and the molar ratio of the amino groups in the polymer compound is 0.5 mol or higher per mol of the drug, more preferably, the amount to be used of the polymer compound is 25% or less and the molar ratio of the amino groups is from 0.5 to 10 mol, and still preferably the amount to be used of the polymer compound is 10% or less and the molar ratio of the amino groups is from 0.5 to 5 mol. When the polymer compound having amino groups is contained at 50% or more, physical property of the preparation remarkably decreases and becomes unfavorable as the transdermal preparation.

Even when a low molecular weight compound having tertiary amino groups such as meglumine and triethanol amine is used, similar results are obtained for the desalt of the drug and the formation of ion pairs . But, the skin irritation is concerned for these compounds per se, which are not preferable as the transdermal preparation in some cases. On the contrary, when using the polymer compound having amino groups, its migration to the skin is extremely low and its safety for the skin is high.

Examples of carboxylic acid used in the invention include, aliphatic monocarboxylic acids such as acetic acid, propionic acid, isobutyric acid, hexanoic acid, octanoic acid, decanoic acid, lauric acid, myristic acid, palmitic acid, stearic acid and isostearic acid, aliphatic dicarboxylic acids such as oxalic acid, succinic acid and glutaric acid, aliphatic unsaturated carboxylic acids such as oleic acid, linoleic acid, linolenic acid, maleic acid and fumaric acid, oxycarboxylic acids such as citric acid, lactic acid, tartaric acid and salicylic acid, ketocarboxylic acids such as pyruvic acid, aromatic carboxylic acids such as phthalic acid, benzoic acid and acetylsalicylic acid, and steroid carboxylic acids such as cholic acid, deoxycholic acid and dehydrocholic acid.

Also, salts of these carboxylic acids include, for example, alkali metallic salts such as Na, K and Li, alkali earth metallic salts such as Ca, Ba and Mg, and ammonium salts or the like.

Among these carboxylic acids and/or the salts thereof, more preferred are aliphatic monocarboxylic acids and oxycarboxylic acids, and in particular, acetic acid, propionic acid, lactic acid and salicylic acid are preferable.

These carboxylic acids and/or the salts thereof may be used alone or in combination with two or more.

As the amount to be used of these carboxylic acids and/or the salts thereof, it is desirable to formulate preferably from 0.01 to 20% , more preferably from 0.1 to 15%, and still preferably from 0.1 to 10% by weight based on the whole amount of the adhesive layer of the adhesive pharmaceutical preparation, in consideration of the sufficient permeated amount and irritation to the skin as the transdermal preparation, inter alia, the adhesive pharmaceutical preparation. Also, a formulation ratio of the drug forming the acid addition salt to the carboxylate salt is preferably from 5/1 to 1/10 (molar ratio).

The drugs used in the invention are not particularly limited as long as the drug forms the acid addition salt, and include, for example, soporific/sedatives (flurazepam hydrochloride, rilmazafone hydrochloride, etc.), antifebrile antiflash analgesic agents (butorphanol tartrate, perisoxal citrate, etc.), stimulant/awakening agents (methanephetamine hydrochloride, methylphenidate hydrochloride, etc.), psychoneurologic agents (sertraline hydrochloride, fluvoxamine maleate, etc.), local anesthetics (lidocaine hydrochloride, procaine hydrochloride, etc.), urological agents (oxybutynin hydrochloride, etc.), skeletal muscle relaxants (tizanidine hydrochloride, eperisone hydrochloride, pridinol mesilate, etc.), autonomic drugs (carpronium chloride, neostigmine bromide, etc.), anti-Parkinson agents (pergolide mesilate, bromocriptine mesilate, trihexiphenydil hydrochloride, amantadine hydrochloride, etc.), anti-histamine agents (clemastine fumarate, diphenhydramine tannate, etc.), bronchodilators (tulobuterol hydrochloride, procaterol hydrochloride, etc.), cardiotonic drugs (isoprenaline hydrochloride, dopamine hydrochloride, etc.), coronary vasodilator drugs (diltiazem hydrochloride, varapamil hydrochloride, etc.), peripheral vasodilator drugs (nicametate citrate, tolazoline hydrochloride, etc.), cardiovascular drugs (flunarizine hydrochloride, nicardipine hydrochloride, benidipine hydrochloride, efonidipine hydrochloride, etc.), drugs for arrhythmia (propranorol hydrochloride, alprenolol hydrochloride, etc.), anti allergic drugs (ketotifen fumarate, azelastine hydrochloride, etc.), antidinics (betahistine mesilate, difenidol hydrochloride, etc.), serotonin receptor antagonistic antiemetics, and narcotic analgesics (morphine sulfate, fentanyl citrate, etc.).

These drugs may be used alone or in combination with two or more, and of course include either forms of inorganic salts or organic salts. Its amount to be used is desirably formulated at the amount of from 0.1 to 50% by weight based on the whole amount of the adhesive layer, in consideration of the sufficient permeated amount as the adhesive pharmaceutical preparation and its irritation to the skin such as rubefaction.

A penetration enhancer may be included in the adhesive layer of the adhesive pharmaceutical preparation of the invention in addition to the polymer compound having amino groups, the drug forming the acid addition salt, and carboxylic acid and/or the salt thereof. The penetration enhancer capable of being used include all compounds of which enhancement action has been conventionally observed in the skin. That is, included are aliphatic alcohols, aliphatic esters, aliphatic amides, aliphatic ethers, aromatic alcohols, aromatic carboxylate esters or ethers (may be either saturated or unsaturated, and either cyclic, linear or branched), and further, monoterpene compounds, sesquiterpene compounds, Azone, Azone derivatives, glycerol fatty acid esters, propyleneglycol fatty acid esters, sorbitan fatty acid esters (Span), polysorbate (Tween), polyethyleneglycol fatty acid esters, polyoxyethylene hydrogenated caster oils (HCO), polyoxyethylene alkyl ethers, sucrose fatty acid esters, vegetable oils and the like.

Specific examples of them include, for example, lauryl alcohol, myristyl alcohol, oleyl alcohol, isostearyl alcohol, cetyl alcohol, methyl laurate, hexyl laurate, diethanolamide laurate, isopropyl myristate, myristyl myristate, octyl dodecyl myristate, cetyl palmitate, salicylic acid, methyl salicylate, ethyleneglycol salicylate, methyl cinnamate, cresol, cetyl lactate, lauryl lactate, ethyl acetate, propyl acetate, geraniol, thymol, eugenol, terpinerol, 1-menthol, bornerol, d-limonene, isoeugenol, isobornerol, nerol, dl-camphor, glycerine monocaprylate, glycerine monocaprate, glycerine monolaurate, glycerine monooleate, sorbitan monolaurate, sucrose monolaurate, polysorbate 20, propyleneglycol, propyleneglycol monolaurate, polyethyleneglycol monolaurate, polyethyleneglycol monostearate, polyoxyethylene lauryl ether, HCO-60, pirotiodecane, olive oil and the like. In particular, preferred are lauryl alcohol, isostearyl alcohol, diethanolamide laurate, glycerine monocaprylate, glycerine monocaprate, glycerine monooleate, sorbitan monolaurate, propyleneglycol monolaurate, polyoxyethylene lauryl ether, pirotiodecane and the like.

Such penetration enhancer may be used in combination with two or more. It is desirable that the amount to be used is formulated at preferably from 0.01 to 20% by weight, and more preferably from 0.05 to 10% by weight, still more preferably from 0.1 to 5% by weight based on the whole amount of the adhesive layer, in consideration of sufficient permeability as the adhesive pharmaceutical preparation and irritation to the skin such as rubefaction and edema.

A plasticizer may be contained in the adhesive layer of the transdermal preparation of the invention. The plasticizers used include petroleum oils (e.g., liquid paraffin, paraffin process oils, naphthene process oils, aromatic process oils, etc.), squalane, squalene, vegetable oils (e.g., olive oil, camellia oil, caster oil, tall oil, peanut oil), silicon oil, dibasic acid esters (e.g., dibutyl phthalate, dioctyl phthalate, etc.), liquid gums (e.g., liquid polybutene, liquid isoprene gum), liquid fatty acid esters (isopropyl myristate, hexyl laurate, diethyl sebacate, diisopropyl sebacate), diethyleneglycol, polyethyleneglycol, glycol salicylate, propyleneglycol, dipropyleneglycol, triacetine, triethyl citrate, crotamiton and the like. In particular, preferred are liquid paraffin, liquid polybutene, crotamiton, diethyl sebacate, hexyl laurate, and the like.

These plasticizers may be used in combination with two or more. It is desirable that the amount to be formulated of such a plasticizer is from 10 to 70%, preferably from 10 to 60%, and still preferably from 10 to 50% by weight in total based on the whole amount of the adhesive preparation layer, in consideration of sufficient permeability and retention of aggultinability as the adhesive pharmaceutical preparation.

Rubber polymer is typically used for the basis of the adhesive layer of the adhesive pharmaceutical preparation of the invention. Specific examples of the rubber polymers include stylene-isoprene-stylene block copolymer (abbreviated as SIS, hereinafter), isoprene gum, polyisobutyrene (abbreviated as PIB, hereinafter), stylene-butadiene-stylene block copolymer (abbreviated as SBS, hereinafter), stylene-butadiene rubber (abbreviated as SBR, hereinafter) and the like. SIS and PIB are preferable, and SIS is especially preferred.

Such rubber polymers may be used in combination with two or more. It is desirable that the amount to be formulated of these rubber polymers is from 5 to 60%, preferably from 10 to 50%, and more preferably from 20 to 40% by weight based on the whole amount of the adhesive layer, in consideration of the formation of the adhesive layer and sufficient skin permeability of the drug.

As the basis of the adhesive layer of the invention, (meth)acrylate polymers (copolymer containing at least one of 2-methylhexyl acrylate, methyl (meth)acrylate, butyl (meth)acrylate, hydroxyethyl (meth)acrylate, glycidyl (meth)acrylate and (meth)acrylic acid) can be used as needed. Such acrylate polymers may be used in combination with two or more or with the rubber polymer described above. It is desirable that the amount to be formulated of these acrylate polymers is from 2 to 88%, preferably from 5 to 50%, and more preferably from 10 to 40% by weight based on the whole amount of the adhesive layer, in consideration of the formation of the adhesive layer and sufficient skin permeability of the drug.

Tackifying resin may be further contained in the adhesive layer of the adhesive pharmaceutical preparation of the invention. The tackifying resins capable of being used include rosin derivatives (e.g., rosin, glycerine ester of rosin, hydrogenated rosin, glycerine ester of hydrogenated rosin, pentaerythritol ester of rosin, etc.), alicyclic saturated hydrocarbon resins [e.g., Arkon P100 (brand name, Arakawa Chemical Industries Ltd.)], aliphatic hydrocarbon resins [e.g., Quinton B170 (brand name, Zeon Corporation)], terpene resins [e.g., Clearon P-125 (brand name, Yasuhara Chemical Co., Ltd)], maleate resins and the like. In particular, glycerine esters of hydrogenated rosin, alicyclic hydrocarbon resins, aliphatic hydrocarbon resins and terpene resins are preferred.

It is desirable that the amount to be formulated of such tackifying resins is from 5 to 10%, preferably from 5 to 60%, and more preferably from 10 to 50% by weight based on the whole amount of the adhesive layer, in consideration of sufficient adhesion as the adhesive pharmaceutical preparation and irritation to the skin at a peeling time.

Also, an antioxidant, filler, crosslinker, preservative, UV absorber and the like can be used for the adhesive layer of the adhesive pharmaceutical preparation of the invention as needed.

Preferable antioxidants include tocopherol and ester derivatives thereof, ascorbic acid, ascorbate stearate ester, nordi hydroguaiaretic acid, dibutylhydroxy toluene (BHT), butylhydroxy anisole and the like.

Preferable fillers include calcium carbonate, magnesium carbonate, silicates (e.g., aluminium silicate, magnesium silicate, etc.), silicic acid, barium sulfate, calcium sulfate, calcium zincate, zinc oxide, titanium oxide and the like.

Preferable crosslinkers include thermoplastic resins such as amino resins, phenol resins, epoxy resins, alkyd resins, and unsaturated polyesters, isocyanate compounds, block isocyanate compounds, organic crosslinkers, inorganic crosslinkers such as metals and metallic compounds.

Preferable preservatives include ethyl paraoxybenzoate, propyl paraoxybenzoate, butyl paraoxybenzoate and the like.

Preferable UV absorbers include p-aminobenzoate derivatives, anthranilate derivatives, salicylate derivatives, coumarin derivatives, amino acid compounds, imidazoline derivatives, pyridine derivatives, dioxane derivatives and the like.

It is desirable that such an antioxidant, filler, crosslinker, preservative, UV absorber and the like are formulated at the sum of preferably 10% or less, more preferably 5% or less, and still preferably 2% or less by weight based on the whole amount of the adhesive layer.

The adhesive layer of the adhesive pharmaceutical preparation of the invention can be also produced by any of methods themselves known in the art. That is, for instance, the present preparation may be obtained by melting the basis ingredient containing the drug with heat and applying onto releasing paper or support followed by being affixed with the releasing paper or the support, or by dissolving the basis ingredient containing the drug in the solvent such as toluene, hexane and ethyl acetate, then extending onto the releasing paper or the support followed by drying and removing the solvent, and subsequently being affixed with the support or the releasing paper.

In the adhesive pharmaceutical preparation of the invention, the materials of the other configuration and each component may be any types as long as the adhesive layer is the above composition containing the polymer compound having amino groups, the carboxylic acid and/or the salt thereof, and the drug.

For instance, the adhesive pharmaceutical preparation of the invention can be made of a backing layer supporting for the above adhesive layer and a releasing paper layer laid the adhesive layer in addition to the above adhesive layer.

The backing layer can use elastic or non-elastic backing. For example, it is selected from fabric, non-woven, polyurethane, polyester, polyvinyl acetate, polyvinylidene chloride, polyethylene, polyethylene terephthalate, aluminium sheet, or composite materials thereof.

That is, as long as the transdermal preparation of the invention is the above composition containing the acid addition salt of the basic drug or amphoteric drug, the polymer compound having amino groups, and the carboxylic acid and/or the salt thereof, the materials of the other configuration and each component may be any of types.

### Examples

The present invention is more specifically illustrated below by examples and test examples, but the invention is not limited to these examples and test examples, and various changes can be made within the scope of the technical thoughts of the invention.

In the examples, all "%" mean % by weight.

| Example 1 | |
|---|---|
| SIS | 20.0% |
| PIB | 10.0 |
| Alicyclic saturated hydrocarbon resin (Arkon P100: brand name, Arakawa Chemical Industries Ltd.) | 38.0% |
| Liquid paraffin | 20.0% |
| Methyl methacrylate-butyl methacrylate-dimethylaminoethyl methacrylate copolymer (Eudragit E100: brand name, Rohm GmbH) | 5.0% |
| Acetic acid | 1.0% |
| Procaterol hydrochloride | 3.0% |
| Pirotiodecane | 3.0% |
| Total | 100.0% |

Precedently, procaterol hydrochloride, acetic acid, pirotiodecane and liquid paraffin were placed in a mortar and mixed thoroughly, and subsequently mixed with the remaining ingredients dissolved in toluene. The mixture was applied on releasing paper followed by drying/removing the solvent, and was affixed with a backing to afford the matrix adhesive pharmaceutical preparation of the invention.

| Example 2 | |
|---|---|
| SIS | 10.0% |
| Acrylate polymer (Duro-Tak87-2097: brand name, National Starch & Chemical Company) | 23.0% |
| Hydrogenated rosin ester | 25.0% |
| (Ester gum H: brand name, Arakawa Chemical Industries Ltd.) | |
| Liquid paraffin | 15.0% |
| Polyvinyl acetal diethylamino acetate (AEA: brand name, Sankyo Co., Ltd.) | 20.0% |
| Propionic acid | 3.0% |
| Tizanidine hydrochloride | 1.0% |
| Diethanolamide laurate | 3.0% |
| Total | 100.0% |

Precedently, tizanidine hydrochloride, propionic acid, diethanolamide laurate and liquid paraffin were placed in a mortar and mixed thoroughly, and subsequently mixed with the remaining ingredient dissolved in ethyl acetate. The mixture was applied on releasing paper followed by drying/removing the solvent, and was affixed with a backing to afford the matrix adhesive pharmaceutical preparation of the invention.

| Example 3 | |
|---|---|
| Acrylate polymer (Duro-Tak387-2287: brand name, National Starch & Chemical Company) | 30.0% |
| Terpene resin (Clearon P125: brand name, Yasuhara Chemical Co., Ltd.) | 10.0% |
| Isopropyl myristate | 10.0% |
| Eudragit E100 (brand name, Rohm GmbH) | 40.0% |
| Lactic acid | 2.0% |
| Pridinol mesilate | 5.0% |
| Glyceryl monocaprylate | 3.0% |
| Total | 100.0% |

Precedently, pridinol mesilate, lactic acid, glycerine monocaprylate and isopropyl myristate were placed in a mortar and mixed thoroughly, and subsequently mixed with the remaining ingredients dissolved in ethyl acetate. The mixture was applied on releasing paper followed by drying/removing the solvent, and was affixed with a backing to afford the matrix adhesive pharmaceutical preparation of the invention.

| Example 4 | |
|---|---|
| PIB | 30.0% |
| Alicyclic saturated hydrocarbon resin (Arkon P100: brand name, Arakawa Chemical Industries Ltd.) | 30.0% |
| liquid paraffin | 20.0% |
| Chitosan | 5.0% |
| Salicylic acid | 7.0% |
| Oxybutynin hydrochloride | 5.0% |
| Glyceryl monocaprate | 3.0% |
| Total | 100.0% |

Precedently, oxybutynin hydrochloride, salicylic acid, glycerine monocaprate and liquid paraffin were placed in a mortar and mixed thoroughly, and subsequently mixed with the remaining ingredients dissolved in ethyl acetate. The mixture was applied on releasing paper followed by drying/removing the solvent, and was affixed with a backing to afford the matrix adhesive pharmaceutical preparation of the invention.

| Example 5 | |
|---|---|
| SIS | 4.7% |
| Acrylate polymer (Duro-Tak387-2287: brand name, National Starch & Chemical Company) | 11.0% |
| Alicyclic hydrocarbon resin | 40.0% |
| Liquid paraffin | 15.0% |
| Eudragit E100 (brand name, Rohm GmbH) | 9.5% |
| Acetic acid | 4.0% |
| Sodium acetate | 1.8% |
| Pergolide mesilate | 9.0% |
| Isostearyl alcohol | 3.0% |
| Sorbitan monolaurate | 2.0% |
| Total | 100.0% |

Precedently, pergolide mesilate, acetic acid, sodium acetate, isostearyl alcohol, sorbitan monolaurate and liquid paraffin were placed in a mortar and mixed thoroughly, and subsequently mixed with the remaining ingredients dissolved in the mixed solvent of ethyl acetate and hexane. The mixture was applied on releasing paper followed by drying/removing the solvent, and was affixed with a backing to afford the matrix adhesive pharmaceutical preparation of the invention.

| Example 6 | |
|---|---|
| SIS | 11.9% |
| Acrylate polymer (Duro-Tak387-2287: brand name, National Starch & Chemical Company) | 5.1% |
| Alicyclic hydrocarbon resin | 35.0% |
| Liquid paraffin | 15.0% |
| Eudragit E100 (brand name, Rohm GmbH) | 12.7% |
| Pirotiodecane | 3.0% |
| Sodium acetate | 5.9% |
| Acetic acid | 1.4% |
| Ambroxol hydrochloride | 10.0% |
| Total | 100.0% |

Precedently, ambroxol hydrochloride, acetic acid, sodium acetate, pirotiodecane and liquid paraffin were placed in a mortar and mixed thoroughly, and subsequently mixed with the remaining ingredients dissolved in the mixed solvent of ethyl acetate and toluene. The mixture was applied on releasing paper followed by drying/removing the solvent, and was affixed with a backing to afford the matrix adhesive pharmaceutical preparation of the invention.

### Comparative examples 1 to 6

Except for excluding the polymer compound having amino groups, the same composition ingredients and the same preparation method as those in respective examples 1 to 6 were used to prepare the corresponding matrix adhesive pharmaceutical preparation, which rendered the comparative examples 1 to 6, respectively.

### Comparative examples 7 to 12

Except for using triethanolamine instead of the polymer compound having amino groups, the same composition ingredients and the same preparation method as those in respective examples 1 to 6 were used to prepare the corresponding matrix adhesive pharmaceutical preparation, which rendered the comparative examples 7 to 12, respectively. The amount of triethanolamine added was so as to be identical to the mol of the amino groups in the polymer compound.

### Comparative examples 13 to 18

Except for containing 55% of the polymer compound having amino groups in the examples 1 to 6, the same composition ingredients and the same preparation method as those in respective examples 1 to 6 were used to prepare the corresponding matrix adhesive pharmaceutical preparation, which rendered the comparative examples 13 to 18, respectively.

### Comparative examples 19 to 22

Except for excluding the carboxylic acid and/or the salt thereof, the same composition ingredients and the same preparation method as those in respective examples 1 to 4 were used to prepare the corresponding matrix adhesive pharmaceutical preparation, which rendered the comparative examples 19 to 22, respectively.

### Test example 1: Skin permeation test in hairless mice

Back skin of a hairless mouse was peeled, and the skin at a dermal side directed to a receptor layer side was loaded in a flow through cell (5 cm²) where warmed water at 37°C was circulated at its periphery. The preparation obtained in the examples 1 to 5, the comparative examples 1 to 5 and 16 to 20 was applied on the side of stratum corneum, saline was used at the side of the receptor layer, and sampling at a rate of 5 ml/hr was carried out every one hour for 18 hours. A flow amount of the receptor solution obtained every one hour was measured accurately, a drug concentration was measured by a high performance liquid chromatography, a permeation rate per hour was calculated, and a skin permeation rate per unit area at a steady state was determined. The result is shown in Table 1.

**Table 1.**

| Example | Drug skin permeation rate | Comparative example | Drug skin permeation rate | Comparative example | Drug skin permeation rate |
|---|---|---|---|---|---|
| | (µg/cm²/hr) | | (µg/cm²/hr) | | (µg/cm²/hr) |
| 1 | 2.8 | 1 | 0.1 | 19 | 1.0 |
| 2 | 5.8 | 2 | 0.3 | 20 | 2.2 |
| 3 | 3.2 | 3 | 0.2 | 21 | 0.8 |
| 4 | 7.2 | 4 | 0.1 | 22 | 1.2 |
| 5 | 6.5 | 5 | 2.1 | | |
| 6 | 33.0 | 6 | 17.0 | | |

As is obviously shown in Table 1, it is found that the drug skin permeation rate is extremely high in the preparation obtained in each example of the present invention containing both the polymer compound having amino groups and the carboxylic acid and/or the salt thereof compared to those in the preparations obtained in the comparative examples 1 to 6 containing only the carboxylic acid and/or the salt thereof and in the preparations obtained in the comparative examples 19 to 22 containing only the polymer compound having amino groups.

### Test example 2: Skin primary irritation test in rabbits

Skin primary irritation of the preparations obtained in the example 1 to 6 and the comparative examples 7 to 12 were examined by Draize method. The resultant PII value in each preparation is shown in Table 2.

**Table 2**

| Example | PII value | Comparative example | PII value |
|---|---|---|---|
| 1 | 0.8 | 7 | 2.5 |
| 2 | 0.9 | 8 | 2.3 |
| 3 | 0.5 | 9 | 3.0 |
| 4 | 0.3 | 10 | 2.1 |
| 5 | 0.7 | 11 | 3.6 |
| 6 | 0.5 | 12 | 2.5 |

As is obviously shown in Table 2, it is found that the skin irritation is extremely low in the preparation obtained in each example of the invention containing the polymer compound having amino groups compared to the preparations obtained in the comparative examples 7 to 12 containing low molecular weight triethanolamine.

### Test example 3: Physical property test in the preparations

Adhesive power was measured using a probe tack tester [brand name: Probe Tack Tester (Rigaku Kogyo KK)] and a peel measuring machine [brand name: Tensilon RTM-100 (KK Orientech)] and cohesive power was measured using a creep measuring machine [brand name: Tensilon RTM-100 (KK Orientech)] for the preparations obtained in the examples 1 to 6 and the comparative examples 13 to 18. Stringiness and leaking of solution components were visually determined. The results were evaluated by assigning those having no problem as ○ and those having problem(s) as × in the preparation physical property. The results are shown in Table 3.

**Table 3**

| Example | Preparation physical property | Comparative example | Preparation physical property |
|---|---|---|---|
| 1 | ○ | 13 | × |
| 2 | ○ | 14 | × |
| 3 | ○ | 15 | × |
| 4 | ○ | 16 | × |
| 5 | ○ | 17 | × |
| 6 | ○ | 18 | × |

As is obviously shown in Table 3, it is found that the preparation physical property was sufficiently sensible in the preparation obtained in each example of the invention in which the content of the polymer compound having amino groups is 50% or less, whereas the preparation physical property is impossibly endurable for the actual use in the preparations obtained in the comparative examples 13 to 18 wherein the polymer compound having amino groups was contained at 55%.

### Industrial applicability

The adhesive pharmaceutical preparation of the invention can make the drug be efficiently absorbed in circulating blood through the skin since its drug skin permeation rate is remarkably high. Also, gastrointestinal side effects and central nervous side effects which can occur with rapid elevation of blood levels observed in the case of the oral administration can be avoided. Additionally, its irritation to the skin is extremely low and it is excellent in physical stability. Thus, it is especially useful as the external preparation for the purpose of percutaneous application.

## Claims

1. A transdermal preparation containing a polymer compound having amino groups, a drug forming an acid addition salt, and carboxylic acid and/or a salt thereof, **characterized in that** the content of the polymer compound having amino groups is 50% or less by weight based on the whole amount of the preparation, a molar ratio of the amino groups in the polymer compound is 0.5 mol or higher per mol of the drug, and the content of the carboxylic acid and/or the salt thereof is 1 to 10 mol per mol of the sum of the drug and amino groups contained in the polymer compound.

2. A transdermal preparation according to claim 1, wherein the polymer compound having amino groups is the polymer compound having primary amino groups.

3. The transdermal preparation according to claim 2, wherein the polymer compound having the primary amino groups is chitosan.

4. The transdermal preparation according to claim 1, wherein the polymer compound having amino groups is the polymer compound having tertiary amino groups.

5. The transdermal preparation according to claim 4, wherein the polymer compound having tertiary amino groups is methyl methacrylate-butyl methacrylate-dimethylaminoethyl methacrylate copolymer or polyvinyl acetal diethylamino acetate.

6. The transdermal preparation according to any of claims 1 to 5, wherein the carboxylic acid is acetic acid, propionic acid, lactic acid and/or salicylic acid.

7. An adhesive pharmaceutical preparation comprising the transdermal preparation according to any of claims 1 to 6.

8. The adhesive pharmaceutical preparation according to claim 7, which is a non-aqueous tape preparation.

9. The adhesive pharmaceutical preparation according to claim 7 or 8 containing a rubber polymer compound in a basis of an adhesive layer.

10. The adhesive pharmaceutical preparation according to claim 9, wherein the rubber polymer compound is stylene-isoprene-stylene block copolymer.

11. A method for improving skin permeability and skin irritation of the transdermal preparation comprising a drug forming an acid addition salt, **characterized by** containing a polymer compound having amino group and carboxylic acid and/or a salt thereof.

12. The method for improving according to claim 11, wherein these compounds are used such that the content of the polymer compound having amino groups is 50% or less by weight based on the whole amount of the preparation, the molar ratio of the amino groups in the polymer compound is 0.5 mol or higher per mol of the drug, and the content of the carboxylic acid and/or the salt thereof is 1 to 10 mol per mol of the sum of the drug and the amino groups contained in the polymer compound.

13. The method for improving according to claim 12, wherein the polymer compound having amino groups is the polymer compound having primary amino groups and the carboxylic acid is acetic acid, propionic acid, lactic acid and/or salicylic acid.

14. The method for improving according to claim 13, wherein the polymer compound having primary amino groups is chitosan.

15. The method for improving according to claim 12, wherein the polymer compound having amino groups is the polymer compound having tertiary amino groups and the carboxylic acid is acetic acid, propionic acid, lactic acid and/or salicylic acid.

16. The method for improving according to claim 15, wherein the polymer compound having tertiary amino groups is methyl methacrylate-butyl methacrylate-dimethylaminoethyl methacrylate copolymer or polyvinyl acetal diethylamino acetate.
